# EUROPEAN PATENT SPECIFICATION

(11) **EP 3 028 646 B1**
(45) Date of publication and mention of the grant of the patent: **27.03.2019**
(21) Application number: 15197288.2
(22) Date of filing: 01.12.2015
(51) Int. Cl.: A61B 10/00

(54) **FOLDABLE STOOL SAMPLE COLLECTION DEVICE**
FALTBARE STUHLPROBENENTNAHMEVORRICHTUNG
DISPOSITIF DE COLLECTE D'ÉCHANTILLONS DE SELLES PLIABLE

(30) Priority: 03.12.2014 NL 2013909
(43) Date of publication of application: 08.06.2016
(73) Proprietor: Daklapack Europe B.V., 8218 MA Lelystad (NL)
(72) Inventor: KLAASSEN, Dave Willem, 3853 ME ERMELO (NL)
(74) Representative: EP&C

(56) References cited:
- WO-A1-2008/080220
- DE-U1- 29 921 867
- US-A- 5 463 782
- US-A1- 2011 270 125

## Description

The invention relates to a foldable stool sample collection device, which in use is mounted on a toilet seat for receiving a stool sample from a person.
Collecting a stool sample of a person may be required for determining the medical condition of that person. For this purpose, stool sample collecting devices have been provided which enable collecting a stool sample of a person when that person uses a toilet. This type of stool sample collecting devices is for example known from EP 2 271 266 and WO2008/080220. Typically, these stool sample collecting devices are mounted on the toilet seat and/or the rim of the toilet bowl, such that they provide a platform in the toilet bowl for receiving the stool sample of the person sitting on the toilet seat. Using a stool sample collecting device allows for receiving the stool sample such that a monster can be taken from the stool sample for medical examination. Typically, after the monster has been taken, the remaining stool sample is flushed and the stool sample collecting device is discarded.
Preferably, the stool sample collecting device is a low cost device that can easily discarded after use. Therefore, a stool sample collecting device is preferably made out of a strip of foldable material. These types of stool sample collection devices are known in the prior art, for example from US 2011/0270125 or US 5 463 782, which disclose stool sample collection devices according to the preamble of claim 1. It is however difficult to provide a stable platform that will actually receiving faecal matter using only a strip of foldable material. Also, the receiving area of a stool sample collecting device should be big enough to actually receive the desired material, while at the same time it should be small enough to prevent urine to spoil the sample. Furthermore, it should be easy for a user to mount the stool sample collecting device such that the collecting device provides a stable platform that prevents a received sample from falling down into the toilet bowl.
To receive faecal matter with none or only a limited amount of urine, known devices for receiving faecal matter are often mounted in the rear end of the toilet bowl. Because the device does not extend into the front part of the toilet bowl, the chance of urine mixing with the stool sample is reduced. However, such a lay out of the collecting device increases the risk that a stool sample distributed towards the front of the toilet bowl missed the collecting device or falls from the collecting device.

It is an object of the present invention to provide an alternative foldable stool sample collection device, more in particular to provide a foldable stool sample collection device that combines providing a stable platform for receiving a stool sample in combination with an improved chance of collecting the sample.

The invention therefore provides a foldable stool sample collection device according to claim 1.

A foldable stool sample collection device according to the invention is to be mounted on a toilet seat of a toilet for receiving a stool sample from a person. The foldable stool sample collecting device comprises an elongate sheet of foldable material and is provided with multiple adhesive pads for securing the elongate sheet to a toilet seat.

The elongate sheet extends in a longitudinal direction between a front end and a rear end thereof, and has a stool sample collecting area located between that front end and that rear end of the elongate sheet.

The elongate sheet comprises a central strip and two lateral strips, which central strip extends in the longitudinal direction of the elongate sheet, and which lateral strips are located adjacent to and adjoining the central strip, and wherein a fold is present in the elongate sheet as a joint between each lateral strip and the central strip.

The central strip extends from the front end of the elongate sheet in a backward direction past the stool sample collecting area, and the two lateral strips each extend from the rear end of the elongate sheet in a forward direction past the stool sample collecting area.

The elongate sheet of foldable material comprises a urine pass through area between the front end of the elongate sheet and the stool sample collecting area, in which urine pass through area the elongate sheet of foldable material is provided with one or more urine pass through openings.

At least one of the multiple adhesive pads is provided on a bottom side of the central strip near the front end of the elongate sheet, and at least one of the multiple adhesive pads is provided on a bottom side of each of the lateral strips neat the rear end of the elongate sheet.

The elongate sheet has a length such that, when the collection device is secured with the front end of the elongate sheet to a front part of the toilet seat and with the rear end of the elongate sheet to a back part of a toilet seat, the stool sample collecting area is supported below the toilet seat in the bowl of the toilet.

Thus, the foldable stool sample collection device is an elongate sheet of foldable material which in use is mounted with its front end and rear end on the front part and the back part of the toilet seat respectively. When mounted, the elongate sheet of foldable material thus extends from the front of the toilet bowl up to the rear of the toilet bowl. The chance of a specimen being missed, because the specimen is dropped in front of the collecting device, is thus reduced compared to stool sample collecting devices known form the prior art.

*To provide the elongate sheet of foldable material with the required stability the central strip is at it its front end mounted on the front part of the toilet seat, and the two lateral strips, located on opposite sides of the central strip, are at their rear end mounted on the rear end of the toilet seat.* In this way, the two lateral strips provide the central strip with lateral stability, preventing the weight of a stool sample placed towards the sides of the stool sample collecting area from twisting the elongate sheet and subsequently slipping from the stool sample collecting device into the toilet bowl.

Furthermore, between the lateral strips and the central strip are provided folds which enable the lateral strips to turn upwards relative to the central strip and thus provide the stool sample collecting device, when in uses, with a U-shaped cross section, when seen in its longitudinal direction. This feature provides additional stability to the elongate sheet of foldable material. Furthermore, the upstanding lateral strips reduce the chance of the stool sample sliding from the stool sample collecting device into the toilet bowl.

Furthermore, the central strip is provided with multiple openings for passing through urine. Thus the strip provides the stool sample collecting device with stability and an enhanced chance of collecting a stool sample, while allowing urine to pass through the collecting device to prevent it from mixing with a collected stool sample.

According to the invention, a stool sample collection device can thus be made from a single sheet of foldable material, and still provide a stable platform for receiving a stool sample from a person in combination with an improved chance of collecting the sample, .i.e. reduce the chance of the sample sliding from the collecting device into the toilet bowl. The use of a sheet material allows for the stool sample collecting device to simply be folded into a flat an compact configuration, for example for transport by mail inside an envelope. There is no need for different components that have to be separated to allow for a compact configuration during transport, and that are to be combined into a single device prior to mounting the device on a toilet bowl.

Preferably, the multiple adhesive pads for mounting the elongate seat of the collecting device onto the toilet seat are already attached to the stool sample collecting device, more in particular to the bottom side of the elongate sheet, during the manufacturing process. Thus, the multiple adhesive pads are already attached to the stool sample collecting device, more in particular to the bottom side of the elongate sheet, when the stool sample collecting device is presented to the user for mounting it on the toilet seat. In an alternative embodiment, the user is presented with an elongate sheet and separate adhesive pads, for example in the form of double sided tape, which the user attaches to the elongate sheet when mounting the sheet on a toilet sheet. In a preferred embodiment, the preferred positions for attaching the pads to the sheet are marked on the sheet.
Providing the pads separate from the elongate sheet enables the user to decide on the exact position of the pads on the sheet when mounting the elongate sheet on the toilet seat, and thus allows for taking into account the specific shape and/or dimensions of the toilet seat the stool collecting device is to be mounted on.

Because the stool sample collection device according to the invention is made from a sheet of foldable material, it can be folded into a flat and compact shape. A stool sample collection device according to the invention can thus easily be stored and transported, and is especially suitable for mass distribution, for example by mail.

In an embodiment of a stool collecting device according to the invention, the multiple openings in urine pass through area comprises a row of multiple elongated openings, each having a longitudinal axis extending parallel to a longitudinal axis of the elongate sheet. Thus, the pass through area comprises multiple connecting parts, i.e. parts of the elongated sheet that extend between the longitudinal openings and that connect the part of the central strip that in use is mounted on the toilet seat with the part of the central strip for receiving the stool sample. When a stool sample has been received on the stool sample collecting area, these connecting parts thus enable transfer of the weight of the stool sample supported by the collecting device along straight lines to the part of the central strip mounted on the toilet seat, preventing substantial twisting of the elongate sheet. Providing elongate openings that extend in the longitudinal direction of the elongate sheet of foldable material thus combines large openings in the strip with a stable platform for receiving a stool sample.

In a further embodiment, in addition to the elongated openings, the urine pass through area comprises multiple circular shaped openings, preferably provided in a row above the row of elongated openings when in use, i.e. located towards the front of the elongate sheet such that the circular shaped openings are provided between the elongate openings and the front end of the strip.

Preferably, the openings in the urine pass through area are provided with rounded corners to allow for an optimal load transfer from stool collecting area to the front end of the strip when the stool sample collecting device supports a stool sample. The rounded corners thus reduce the chance of peak loads in the elongate sheet of foldable material causing local tearing of the sheet. Tears might cause instability of the sheet and even cause the stool sample to slide form the collecting device.

In an embodiment, the central strip is furthermore provided with a urine drainage area located between the stool sample collecting area and the urine pass through area, which urine drainage area stretches at least along the width of the central strip, and preferably stretches along the width of the lateral strips as well, which drainage area is provided with multiple openings for draining urine flowing along the central strip from the urine pass through are towards the stool sample collecting area. In contrast with the urine pass through area, which is provided with large openings to minimize contact between a flow of urine and the elongate sheet of foldable material, the urine drainage area is provided for preventing urine flowing along the elongate sheet towards the stool sample collecting area, from reaching that area. Thus, when during the stool collecting process, urine comes into contact with the elongate sheet in stead of passing it, and subsequently flows towards the stool collecting area the urine drainage area may prevent the urine from reaching the stool sample collecting area.

In an embodiment, the urine drainage area comprise at least two rows of openings, and the openings of one of the at least two rows are staggered relative to the openings of the other row. Due to the way the stool sample device is mounted on the toilet seat, i.e. with at least one adhesive pad provided near the front end of the central strip, urine running along the elongate sheet will typically run along the longitudinal direction thereof. By providing at least two rows of overlapping openings, urine that flows in the longitudinal direction of the elongate sheet and passes between two openings of the first row of drainage openings, will encounter an opening of the second row of drainage openings. In a further embodiment, more than two rows of overlapping openings can be provided to further reduce the chance of urine reaching the stool sample collecting area.

Because of the function of the urine drainage area, which differs from the function of the urine pass through area, the openings provided in the urine drainage area can be substantially smaller than the openings provided in the urine pass through area. This is beneficial because providing multiple small openings reduces the overall stability during use of the elongate sheet of foldable material less than large openings.
Furthermore, providing the urine drainage area in combination with the urine pass through area allows for providing the urine pass through area with multiple connecting sections, which connecting sections divide the pass through opening into multiple openings to increase the stability of the strip during use in comparison with a strip having a single large pass through opening, without increasing the chance of urine flowing to the sample collecting area via those connecting sections.
Providing the connecting sections in the urine pass through area reduces the overall pass through opening and thus increase the chance of urine not passing through the opening. By providing the urine drainage area, urine flowing along the elongate sheet, more in particular the connecting sections bridging the urine pass through area, more in particular the urine pass through opening, towards the stool sample collecting area will encounter the urine drainage area, via which drainage openings the flow will be passed through the strip into the toilet bowl. Thus the flow of urine will not reach the stool collecting area.
In a preferred embodiment, the urine drainage openings are substantially smaller than the urine pass through openings, preferably each urine drainage openings has an area size of less than 25% of an areas size of a urine pass through openings. Providing small size openings allows for providing a fine mesh, and thus for a improved structural integrity of the strip, i.e. better stability during use of the stool sample collecting device.

In an embodiment, the collection device, prior to use, is folded into a relatively small, compact shape, in which the lateral strips are folded upon a top side of the central strip, along the fold between each of the two lateral strips and the central strip. By thus folding the elongate sheet of foldable material, when the elongate sheet of foldable material is unfolded and mounted on the toilet seat, the side trips will be prone to extend in an upward direction relative to the central strip, which is beneficial for the function of the lateral strips to provide side walls along the stool sample collecting area, which side walls may keep a received stool sample form falling from the elongate sheet of foldable material.

In an embodiment, the central strip has a rear end located between the stool sample collecting area and the rear end of the elongate sheet of foldable material, such that the a elongate sheet of foldable material is essentially Y-shaped. In this embodiment, the lateral strips, the ends of which are in use attached to the toilet seat, extend beyond the rear end of the central strip. Thus, when the device is mounted on the toilet seat, the rear end of the central strip, which is not attached to the toilet seat, is not directly supported by the toilet seat but is indirectly supported by the toilet seat via the lateral strips, which are attached to the toilet seat. Especially when loaded by a stool sample received in the stool sample collecting area, the lateral strips will be forced in an upward direction relative to the central strip, the lateral strips thus forming upstanding side walls along the stool sample collecting area, which side walls are beneficial for the stability of the elongated strip of foldable material and are beneficial in keeping a received stool sample form falling from the elongate sheet of foldable material.
In a further preferred embodiment, the stool sample collecting device is substantially Y-shaped. In this embodiment, the lateral strips extend at an angle to a longitudinal axis of the central strip in the range of 15-60 degrees, for example at an angle of 45 degrees, preferably in the range of 20-40 degrees, for example at an angle of 30 degrees. Thus, the central strip extends along a central axis of the elongate sheet of the foldable material while the lateral strips, in particular the rear ends thereof, are located on opposite sides of the central axis of the elongate sheet of foldable material and extend at an angle with that longitudinal axis. This Y- shaped configuration provides a stable platform and furthermore biases the lateral strips in an upward direction, thus providing walls alongside the central stool sample collecting area on the central strip for keeping a received stool sample from falling of the device in the lateral direction.

In an embodiment, the lateral strips run from the rear end of the elongate sheet of foldable material up to the front end of the elongate sheet of foldable material, such that when in use the collection device is mounted on an oval or circular shaped, when seen in top view, toilet seat the front ends of the lateral strips rest on part of the toilet seat that curves away from the front of the toilet seat. Thus the shape of the toilet seat is used to hold the lateral strips towards the central strip, or in other words push the lateral strips in an upward direction relative to the central strip, providing the device with a substantially U-shaped cross section. The lateral strips thus provide upstanding walls extending along the central strip, which upstanding walls contribute to keeping a received stool sample from falling of the device in the lateral direction.
In a further embodiment, the lateral strips have a narrow mid section, and flare out towards their rear end and towards their front, providing the lateral strips with wide end sections to further provide a stable support and enable to further utilize the shape of a circular or curved toilet seat, when seen in top view, to push or fold the lateral strips in an upward direction relative to the central strip, when seen in cross section, to provide upstanding walls extending along the stool sample collecting area.

In a further embodiment, the front end of the sheet of foldable material is rounded, such that in use it substantially follows the circumference of a circular or oval shaped, when seen in top view, toilet seat.

Preferably, an elongated strip of foldable material according to the invention has a length in the range of 40 cm - 60 cm, more preferably in the range 45 cm - 55 cm for example has a length of 50 cm. Preferably, the elongate strip of foldable material according to the invention has a width in the range of 20 cm - 40 cm, for example has a width of 30 cm.

In an embedment, the elongate sheet of foldable material is made of paper. Preferably, the elongate sheet of foldable material is a laminate comprising multiple sheets of different types of materials, the different materials each providing the device with particular properties, such as enhanced strength, enhanced foldability at least at the area along the fold between the central strip and the lateral strips.

Preferably, the elongate sheet of foldable material is made of biodegradable material. Thus disposal of the device does not pose environmental problems. Preferably, the device is configured of a biodegradable material that allows for flushing the collecting device after use.

It is observed that the a collecting device according to the invention is preferably mounted on the toilet seat, but alternatively can also be mounted on the rim of the toilet bowl.

Advantageous embodiments of the stool sample collecting device according to the invention are disclosed in the subclaims and in the description, in which the invention is further illustrated and elucidated on the basis of a number of exemplary embodiments, of which some are shown in the schematic drawing.

In the drawing
Fig. 1 shows a top view of a stool sample collecting device according to the invention;
Fig. 2 shows a bottom view of the stool sample collecting device of Fig. 1;
Fig. 3 shows a perspective view of the stool sample collecting device of Fig. 1; and
Fig. 4 shows the stool sample collecting device mounted on a toilet seat for receiving a stool sample from a person.

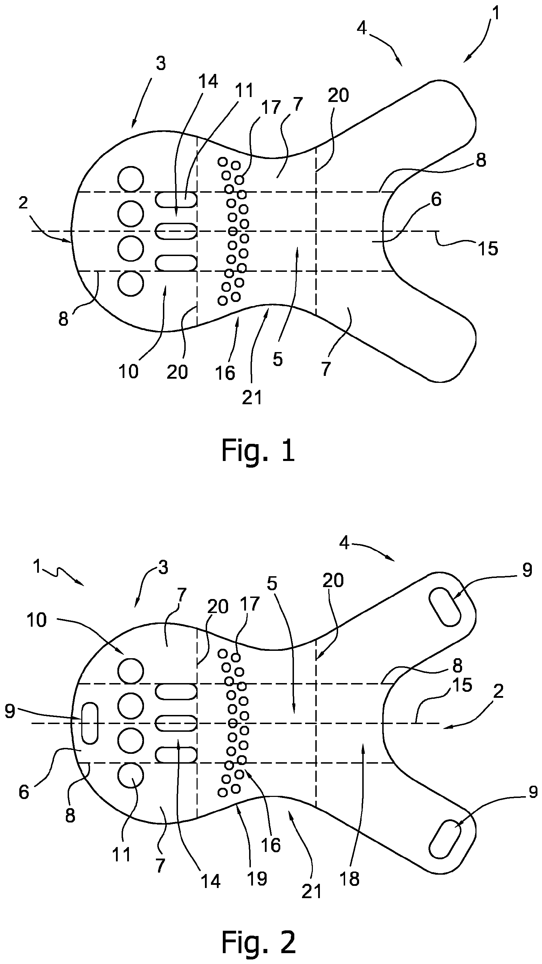

Fig. 1 shows a top view of a stool sample collecting device 1 according to the invention. The foldable stool sample collecting device 1 comprises an elongate sheet 2 of foldable material. The elongate sheet 2 extends in a longitudinal direction between a front end 3 and a rear end 4, and has a stool sample collecting area 5 located between the front end and the rear end for receiving a stool sample from a person.

The elongate sheet of foldable material furthermore comprises a urine pass through area 10 between the front end 3 of the elongate sheet 2 and the stool sample collecting area 5. In the urine pass through area 10 the elongate sheet 2 of foldable material is provided with one or more openings 11 for passing through urine of a person from which a stool sample is being collected.

The elongate sheet 2 comprises a central strip 6 and two lateral strips 7. The central strip 6 extends in the longitudinal direction of the elongate sheet 2, from the front end 3 of the elongate sheet 2 in a backward direction past the stool sample collecting area 5. The lateral strips 7 are located adjacent to and adjoining the central strip 6 and each extend from the rear end 4 of the elongate sheet in a forward direction past the stool sample collecting area 5. A fold 8, indicated with a dotted line, is present in the elongate sheet as a joint between each lateral strip and the central strip.

The folds 8 enables the lateral strips 7 to fold in an upward direction relative to the central strip 6, when the foldable stool sample collecting device is mounted on a toilet seat. Thus, the lateral strips form upstanding walls along the stool sample collecting area of the central strip, to stabilize the platform provided by the elongate strip, and to keep a received specimen from sliding of the stool sample collecting area.

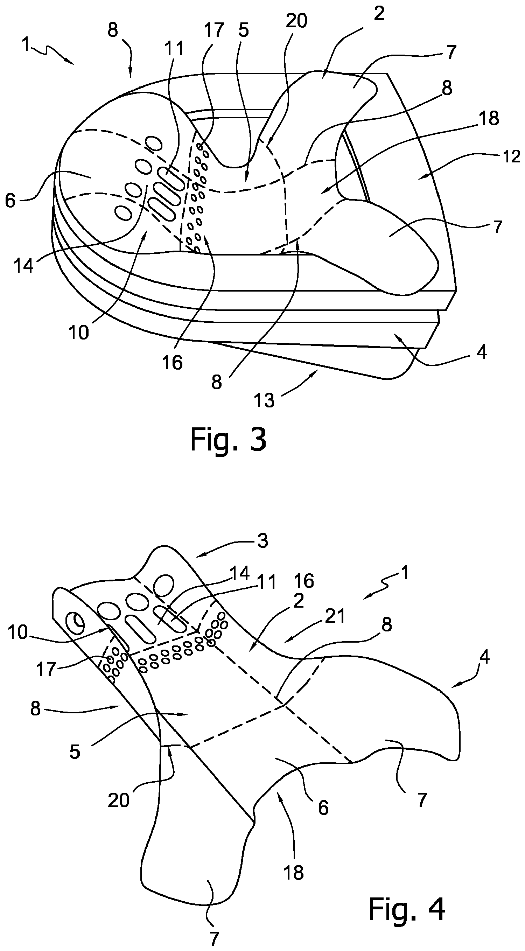

Fig. 3 shows a perspective view of the stool sample collecting device of Fig. 1 with the lateral strips folded upward. It is noted that in use the lateral strips 8 preferably make an angle of at least 30 degrees relative to the central strip 6, more preferably an angle of at least 45 degrees, for example of 60 degrees. Thus, the side strips provide the device with a U-shaped cross section.

The foldable stool sample collecting device 1 is provided with multiple adhesive pads 9 for securing the elongate sheet 2 to a toilet seat. Fig. 2 shows a bottom view of the same stool sample collecting device, in which the adhesive pads are indicated. One of the multiple adhesive pads 9 is provided on a bottom side of the central strip 6 near the front end 3 of the elongate sheet 2. One of the multiple adhesive pads 9 is provided on a bottom side of each of the lateral strips 7 near the rear end of the elongate sheet 2.

The elongate sheet 2 has a length such that, when the collection device 1 is secured with the front end of 3 the elongate sheet to the front part of a toilet seat and with the rear end 4 of the elongate sheet to the back part of a toilet seat, the stool sample collecting area 5 is supported below the toilet seat in the bowl of the toilet.

Preferably, an elongated strip of foldable material according to the invention has a length in the range of 40 cm - 60 cm, more preferably in the range 45 cm - 55 cm for example has a length of 50 cm. Preferably, the elongate strip of foldable material according to the invention has a width in the range of 20 cm - 40 cm, for example has a width of 30 cm.

Fig. 4 shows the stool sample collecting device mounted on a toilet seat 12 of a toilet 13, for receiving a stool sample from a person.

As is clear from Figs. 1-4, the foldable stool sample collection device 1 is an elongate sheet 2 of foldable material which in use is mounted with its front end 3 on the front of the toilet seat 12 and with its rear end 4 and back of the toilet seat 12. When mounted, the elongate sheet of foldable material thus extends from the front of the toilet bowl up to the rear of the toilet bowl.

To provide the elongate sheet 2 of foldable material with the required stability the central strip 6 is at it its front end mounted on the front part of the toilet seat 12, and the two lateral strips 7, located on opposite sides of the central strip, are at their rear end mounted on the rear end of the toilet seat 12. In this way, the two lateral strips 7 provide the central strip 6 with lateral stability, preventing the weight of a stool sample placed towards the sides of the stool sample collecting area 5 from twisting the elongate sheet and subsequently slipping from the stool sample collecting device 1 into the toilet bowl.

Furthermore, the folds 8 provided between the lateral strips 7 and the central strip 6 enable the lateral strips to turn upwards relative to the central strip and thus provide the stool sample collecting device, when in uses, with a U-shaped cross section, when seen in its longitudinal direction (see also fig. 4). This in use provides additional stability to the elongate sheet of foldable material. Furthermore, the upstanding lateral strips 7 keep a received stool sample located on the central strip and reduce the chance that the stool sample slips from the stools ample collecting device 1 into the toilet bowl.

It is also clear from Figs. 1-4 that the multiple openings 11 for passing through urine are, when the stool sample collecting device 1 has been mounted on a toilet seat, provided at the front half of the toilet bowl, preferably in the part of the elongate sheet that curves upwards towards the toilet seat.

In the embodiment shown, the urine pass through area 10 is provided with a row of three elongate openings 11, each having a longitudinal axis extending substantially parallel to a longitudinal axis of the elongate sheet 2. Thus, the urine pass through area comprises two connecting parts 14, i.e. parts of the elongated sheet 2 that extend between the longitudinal openings 11 and that connect the part of the central strip 6 mounted on the toilet seat 12 with the part of the central strip for receiving the stool sample. When a stool sample has been received on the stool sample collecting area 5, these connecting parts 14 thus enable transfer of the weight to the part of the central strip 6 mounted on the toilet seat 12, preventing substantial twisting of the elongate sheet. Providing elongate openings that extend in the longitudinal direction of the elongate sheet of foldable material thus combines large openings in the strip with a stable platform for receiving a stool sample.

In the preferred embodiment shown, the central strip 6 is furthermore provided with a urine drainage area 16 located between the stool sample collecting area 5 and the urine pass through area 10. The urine drainage area 16 of the exemplary embodiment shown stretches at along the width of the central strip 6 and along the width of the lateral strips 7. The drainage area 16 is provided with multiple openings 17 for draining urine flowing along the central strip 6 from the urine pass through area 10 towards the stool sample collecting area 5. In contrast with the urine pass through area 10, which is configured for preventing urine to contact the stool sample collecting area during the stool sample collecting process, the urine drainage area 16 is provided for preventing urine flowing along the elongate strip towards the stool sample collecting area, from reaching that area. Thus, when during the stool collecting process urine comes into contact with the elongate sheet in stead of passing it, and subsequently flows towards the stool collecting area, the urine drainage area may prevent the urine from reaching the stool sample collecting area.

In the exemplary embodiment shown, the urine drainage area 16 comprise two rows 19 of urine drainage openings. The openings of one of the two rows are staggered relative to the openings of the other row, when seen in the longitudinal direction of the sheet. Due to the way the stool sample device is mounted on the toilet seat, i.e. with at least one adhesive pad provided near the front end of the central strip, urine running along the elongate strip will typically run along the longitudinal direction thereof. By providing at least two rows 19 of overlapping urine drainage openings 17, urine that flows in the longitudinal direction of the elongate sheet and passes between two openings of the first row of drainage openings, will encounter an opening of the second row of drainage openings. In an alternative embodiment, more than two rows of overlapping openings can be provided to further reduce the chance of urine reaching the stool sample collecting area.

Because of the function of the urine drainage area differs from the function of the urine pass through area the provided urine drainage openings are substantially smaller than the urine pass through openings. The many small sized urine drainage openings from a fine mesh which is beneficial because it provides better stability during use than providing large openings. Furthermore, providing the urine drainage area 16 allows for providing the urine pass through area 10 with multiple connecting sections 14, which connecting sections divide the pass through opening into multiple openings to increase the stability of the elongate strip during use, without increasing the chance of urine flowing to the sample collecting area via those connecting sections.

By providing the urine drainage area 16, urine flowing along the elongate sheet, more in particular along the connecting sections 14 bridging the urine pass through area 10, towards the stool sample collecting area 5 will encounter the urine drainage area 16, via which drainage openings 17 the flow will be passed through the elongate sheet into the toilet bowl. Thus the flow of urine will not reach the stool collecting area.

In the preferred embodiment shown, the urine drainage openings 17 are substantially smaller than the urine pass through openings 11. Each urine drainage opening 17 has an area size of less than 25% of an areas size of a urine pass through opening 11. Providing these small size openings allows for providing a fine mesh, and thus for a improved structural integrity of the strip, i.e. better stability during use of the stool sample collecting device.

In the exemplary embodiment shown, in addition to the elongated openings, the urine pass through area 10 comprises additional circular shaped openings, provided in a row above the row of elongated openings. In the exemplary embodiment shown, the all openings in the urine pass through area are provided with rounded corners, to allow for an optimal load transfer within the elongate sheet of foldable material, and reduce the chance of peak loads in the elongate sheet of foldable material causing local tearing of the sheet.

In the embodiment shown, the elongate sheet is provided with the folds 8 extending in the longitudinal direction thereof and with folds 20 extending in the lateral direction of the elongate sheet, i.e. substantially perpendicular to the folds 8. The folds are present in the elongate sheet because prior to use, prior to it being mounted on a toilet seat, it is folded into a relatively small, compact shape. When folded, the lateral strips are folded upon a top side of the central strip, along the fold between each of the two lateral strips and the central strip.
By thus folding the elongate sheet of foldable material, when the elongate sheet of foldable material is unfolded and mounted on the toilet seat, the side trips will be prone to extend in an upward direction relative to the central strip, which is beneficial for the function of the lateral strips to provide side walls along the stool sample collecting area, which side walls may keep a received stool sample form falling from the elongate sheet of foldable material. Furthermore, prior to use, the elongate sheet with its lateral strips folded onto the central strip is folded in its longitudinal direction along the lateral folds 20 such that the front and the end of the elongate sheet are folded onto a central section thereof. Thus a compact shape is achieved.

In the preferred embodiment shown, the central strip 6 has a rear end 4 located between the stool sample collecting area 5 and the rear end 4 of the elongate sheet of foldable material 2, such that the a elongate sheet of foldable material is essentially Y-shaped. In this embodiment, the lateral strips 7, the ends of which are attached onto the toilet seat 12, extend beyond the rear end 18 of the central strip 6. In use, see fig. 4, the rear end of the central strip 6 is not directly supported, but is supported via the lateral strips. Especially when loaded by a stool sample received in the stool sample collecting area, the lateral strips will be forced in an upward direction relative to the central strip, which is beneficial for the function of the lateral strips to provide upstanding side walls along the stool sample collecting area, which side walls may are beneficial for the stability of the elongated strip of foldable material and are beneficial in keeping a received stool sample form falling from the elongate sheet of foldable material.

In the preferred embodiment, the lateral strips 7 extend at an angle to a longitudinal axis 15 of the central strip in the range of 15-60 degrees, at an angle of about 30 degrees. Thus, the central strip 6, in particular the front end thereof, extends along a central axis of the elongate sheet of the foldable material while the lateral strips, in particular the rear ends thereof, are located on opposite sides of the central axis of the elongate sheet of foldable material and partially at an angle with the central axis, see figs 1 and 2.
This Y- shaped configuration of the elongate sheet 2 provides a stable platform and furthermore biases the lateral strips 7 in an upward direction relative to the central strip 6, thus providing walls alongside the central stool sample collecting area on the central strip for keeping a received stool sample from falling of the device in the lateral direction.

In the exemplary embodiment shown, the lateral strips 7 run from the rear end 4 of the elongate sheet 2 of foldable material up to the front end 3 of the elongate sheet 4 of foldable material. When in use the collection device 2 is mounted on an oval or circular shaped, when seen in top view, toilet seat 13, such as the one shown in fig. 4, the front ends of the lateral strips7 rest on part of the toilet seat that curves away from the front of the toilet seat. Thus the shape of the toilet seat is forces the lateral strips 7 towards the central strip 6 and thus pushes the lateral strips in an upward direction. Thus, the shape of the toilet seat is used to contribute to the lateral strips providing upstanding walls alongside the central stool sample collecting area of the central strip, thus keeping a received stool sample from falling of the device in the lateral direction.

In in the exemplary embodiment shown, the lateral strips 7 have a narrow mid section 21, and flare out towards their rear end and towards their front end. Thus providing the lateral strips with wide end sections enables to further utilize the shape of a circular or curved toilet seat, when seen in top view, to push or fold the lateral strips in an upward direction relative to the central strip, when seen in cross section, to provide upstanding walls extending along the stool sample collecting area.

Also, in the exemplary embodiment shown, the front end of the sheet of foldable material is rounded, such that in use it substantially follows the circumference of a circular or oval shaped, when seen in top view, toilet seat.

In an embedment, the elongate sheet of foldable material is made of paper. Preferably, the elongate sheet of foldable material is a laminate comprising multiple sheets of different types of materials, the different materials each providing the device with particular properties, such as enhanced strength, enhanced foldability at least at the area along the fold between the central strip and the lateral strips.

Preferably, the elongate sheet of foldable material and the adhesive strips is made of biodegradable material. Thus disposal of the device does not pose environmental problems.

Preferably, the device is configured of a biodegradable material that allows for flushing the collecting device after use.

The invention provides a stool sample collection device to be mounted on a toilet seat for receiving a stool sample from a person, the stool sample collecting device comprises an elongate sheet of foldable material provided with multiple adhesive pads for securing the elongate sheet to the toilet seat. According to the invention, the stool sample collecting device is configured to provide a stable platform for receiving and supporting a stool sample. Therefore, the elongate sheet comprises a central strip and two lateral strips, which central strip extends in the longitudinal direction of the elongate sheet, and which lateral strips are located adjacent to and adjoining the central strip. A fold is present in the elongate sheet as a joint between each lateral strip and the central strip. When the elongate sheet is mounted on a toilet seat, the lateral strips fold towards each other and towards the central strip such that they provide upstanding wall sections along the central strip. Thus, when in use, the lateral strips provide the elongate sheet with stability and provide a restraint for a stool sample received on the central strip, reducing the chance of a stool sample sliding from the receiving device in the lateral direction thereof.

It is observed that the exemplary embodiment shown is mounted on the toilet sea. Alternatively can also be mounted on the rim of the toilet bowl.

### Reference signs

- 01: foldable stool sample collecting device
- 02: elongate sheet
- 03: front end elongate sheet
- 04: rear end elongate sheet
- 05: stool sample collecting area
- 06: central strip
- 07: lateral strips
- 08: folds between central strip and lateral strips
- 09: adhesive pads
- 10: urine pass through area
- 11: openings of urine pass through area
- 12: toilet seat
- 13: toilet
- 14: connecting parts in urine pass through area
- 15: longitudinal axis elongate sheet
- 16: urine drainage area
- 17: drainage openings
- 18: rear end central strip
- 19: row urine drainage openings
- 20: lateral folds
- 21: narrow mid section lateral strips

## Claims

1. Stool sample collection device (1), which is to be mounted on a toilet seat (12) of a toilet (13) for receiving a stool sample from a person, the stool sample collecting device (1) comprising an elongate sheet (2) of foldable material provided with multiple adhesive pads (9) for securing the elongate sheet (2) to the toilet seat (12),
wherein the elongate sheet (2) extends in a longitudinal direction between a front end (3) and a rear end (4) thereof, and has a stool sample collecting area (5) located between that front end and that rear end of the elongate sheet,
**characterized in that**
the elongate sheet (2) comprises a central strip (6) and two lateral strips (7), which central strip (6) extends in the longitudinal direction of the elongate sheet (2), and which lateral strips (7) are located adjacent to and adjoining the central strip (6), and wherein a fold (8) is present in the elongate sheet (2) as a joint between each lateral strip (7) and the central strip (6),
wherein the central strip (6) extends from the front end (3) of the elongate sheet (2) in a backward direction past the stool sample collecting area (5), and the two lateral strips (7) each extend from the rear end (4) of the elongate sheet (2) in a forward direction past the stool sample collecting area (5),
wherein the elongate sheet (2) of foldable material comprises a urine pass through area (10) between the front end (3) of the elongate sheet (2) and the stool sample collecting area (5), in which urine pass through area (10) the elongate sheet of foldable material is provided with one or more urine pass through openings (11),
wherein at least one of the multiple adhesive pads (9) is provided on a bottom side of the central strip (6) near the front end (3) of the elongate sheet (2), and at least one of the multiple adhesive pads (9) is provided on a bottom side of each of the lateral strips (7) near the rear end (4) of the elongate sheet (2),
and wherein the elongate sheet (2) has a length such that, when the collection device (1) is secured with the front end (3) of the elongate sheet (2) to a front part of the toilet seat (12) and with the rear end (4) of the elongate sheet (2) to a back part of a toilet seat (12), the stool sample collecting area (5) is supported below the toilet seat (12) in a bowl of the toilet (13).

2. Collection device according to claim 1, wherein the urine pass through area (10) comprises a row of multiple elongated openings (11), each having a longitudinal axis extending parallel to a longitudinal axis (15) of the elongate sheet (2).

3. Collection device according to claim 1 or to claim 2, wherein the central strip (6) is furthermore provided with a urine drainage area (16) located between the stool sample collecting area (5) and the urine pass through area (1), which urine drainage area stretches at least along the width of the central strip (6), and preferably stretches along the width of the lateral strips (7) as well, which urine drainage area (16) is provided with multiple drainage openings (17) for draining urine flowing along the central strip (6) from the urine pass through area (10) towards the stool sample collecting area.

4. Collection device according to claim 3, wherein the urine drainage area (16) comprise at least two rows of drainage openings (17), and wherein the drainage openings (17) of one of the at least two rows are staggered relative to the drainage openings (17) of the other row.

5. Collection device according to claim 3 or claim 4, wherein the drainage openings (17) of the urine drainage area (16) are substantially smaller than the urine pass through opening (11) of the urine pass through area (10), preferably the area size of each urine drainage openings (17) is at most 0,25 the area size of a urine pass through openings (10).

6. Collection device according to one or more of the preceding claims, wherein the collection device (1), prior to use, is folded into a compact shape, in which the lateral strips (7) are folded upon a top side of the central strip (6), along the folds (8) between each of the two lateral strips (7) and the central strip (6).

7. Collection device according to one or more of the preceding claims, wherein the central strip (6) has a rear end (18) located between the stool sample collecting area (5) and the rear end (4) of the elongate sheet (2) of foldable material, such that the a elongate sheet of foldable material is essentially Y-shaped.

8. Collection device according to claim 7, wherein the lateral strips (7) extend at an angle to a longitudinal axis (15) of the central strip (6) in the range of 15-60 degrees, for example at an angle of 45 degrees, preferably in the range of 20-40 degrees, for example at an angle of 30 degrees.

9. Collection device according to one or more of the preceding claims, wherein the central strip (6) has along its length a constant width in the range of 3 cm - 12 cm, for example has a constant width of 7 cm.

10. Collection device according to one or more of the preceding claims, wherein the lateral strips (7) run from the rear end (4) of the elongate sheet (2) of foldable material up to the front end (3) of the elongate sheet (2) of foldable material.

11. Collection device according to claim 10, wherein the lateral strips (7) have a narrow mid section, and flare out towards their rear end and towards their front end.

12. Collection device according to claim 10 or claim 11, wherein the front end (3) of the elongate sheet (2) of foldable material is rounded, such that, when it is mounted on a toilet seat during use, it substantially matches the circumference of a toilet seat (12).

13. Collection device according to one or more of the preceding claims, wherein the elongate sheet (2) of foldable material is made of paper and/or is made of biodegradable material..

14. Collection device according to one or more of the preceding claims, wherein the elongate sheet (2) of foldable material is a laminate comprising multiple sheets of different types of material.

15. Postal envelope comprising a folded stool sample collecting device according to one or more of the preceding claims.

## Patentansprüche

1. Stuhlproben-Sammelvorrichtung (1), welche auf einem Toilettensitz (12) einer Toilette (13) zu montieren ist, um eine Stuhlprobe von einer Person zu erfassen, wobei die Stuhlproben-Sammelvorrichtung (1) eine längliche Schicht (2) eines faltbaren Materials umfasst, welche mit mehreren Klebepads (9) versehen ist, um die längliche Schicht (2) an dem Toilettensitz (12) zu befestigen,
wobei sich die längliche Schicht (2) in eine Längsrichtung zwischen einem vorderen Ende (3) und einem hinteren Ende (4) davon erstreckt und einen Stuhlproben-Sammelbereich (5) aufweist, welcher zwischen dem vorderen Ende und dem hinteren Ende der länglichen Schicht angeordnet ist,
**dadurch gekennzeichnet,**
**dass** die längliche Schicht (2) einen zentralen Streifen (6) und zwei seitliche Streifen (7) umfasst, wobei sich der zentrale Streifen (6) in der Längsrichtung der länglichen Schicht (2) erstreckt, und wobei die seitlichen Streifen (7) benachbart zu dem zentralen Streifen (6) angeordnet sind und an diesen angrenzen, und wobei eine Falte (8) in der länglichen Schicht (2) als eine Verbindung zwischen jedem seitlichen Streifen (7) und dem zentralen Streifen (6) vorhanden ist,
wobei sich der zentrale Streifen (6) von dem vorderen Ende (3) der länglichen Schicht (2) in einer nach hinten gerichteten Richtung an dem Stuhlproben-Sammelbereich (5) vorbei erstreckt, und wobei sich die zwei seitlichen Streifen (7) jeweils von dem hinteren Ende (4) der länglichen Schicht (2) in eine nach vorn gerichtete Richtung an dem Stuhlproben-Sammelbereich (5) vorbei erstrecken,
wobei die längliche Schicht (2) eines faltbaren Materials einen Urindurchlassbereich (10) zwischen dem vorderen Ende (3) der länglichen Schicht (2) und dem Stuhlproben-Sammelbereich (5) umfasst, wobei die längliche Schicht eines faltbaren Materials in dem Urindurchlassbereich (10) mit einer oder mit mehreren Urindurchlassöffnungen (11) versehen ist,
wobei mindestens einer der mehreren Klebepads (9) auf einer Bodenseite des zentralen Streifens (6) in der Nähe des vorderen Endes (3) der länglichen Schicht (2) vorhanden ist, und wobei zumindest einer der mehreren Klebepads (9) auf einer Bodenseite von jedem der seitlichen Streifen (7) in der Nähe des hinteren Endes (4) der länglichen Schicht (2) vorhanden ist,
und wobei die längliche Schicht (2) eine Länge aufweist, so dass, wenn die Sammelvorrichtung (1) mit dem vorderen Ende (3) der länglichen Schicht (2) an einem vorderen Teil des Toilettensitzes (12) und mit dem hinteren Ende (4) der länglichen Schicht (2) an einem hinteren Teil eines Toilettensitzes (12) befestigt ist, der Stuhlproben-Sammelbereich (5) unter dem Toilettensitz (12) in einer Schüssel der Toilette (13) gehalten wird.

2. Sammelvorrichtung nach Anspruch 1, wobei der Urindurchlassbereich (10) eine Reihe von mehreren länglichen Öffnungen (11) umfasst, welche jeweils eine Längsachse aufweisen, die sich parallel zu einer Längsachse (15) der länglichen Schicht (2) erstreckt.

3. Sammelvorrichtung nach Anspruch 1 oder Anspruch 2, wobei der zentrale Streifen (6) darüber hinaus mit einem Urinabflussbereich (16) versehen ist, welcher zwischen dem Stuhlproben-Sammelbereich (5) und dem Urindurchlassbereich (10) angeordnet ist, wobei sich der Urinabflussbereich zumindest entlang der Breite des zentralen Streifens (6) dehnt und sich vorzugsweise entlang der Breite der seitlichen Streifen (7) dehnt, wobei der Urinabflussbereich (16) auch mit mehreren Abflussöffnungen (17) versehen ist, um einen Urin abfließen zu lassen, welcher entlang des zentralen Streifens (6) von dem Urindurchlassbereich (10) zu dem Stuhlproben-Sammelbereich fließt.

4. Sammelvorrichtung nach Anspruch 3, wobei der Urinabflussbereich (16) mindestens zwei Reihen von Abflussöffnungen (17) umfasst, und wobei die Abflussöffnungen (17) von mindestens einer der mindestens zwei Reihen versetzt relativ zu den Abflussöffnungen (17) der anderen Reihe sind.

5. Sammelvorrichtung nach Anspruch 3 oder Anspruch 4, wobei die Abflussöffnungen (17) des Urinabflussbereichs (16) im Wesentlichen kleiner als die Urindurchlassöffnung (11) des Urindurchlassbereichs (10) sind, wobei vorzugsweise die Fläche von jeder Urinabflussöffnung (17) höchstens 0,25 der Fläche einer Urindurchlassöffnung (10) beträgt.

6. Sammelvorrichtung nach einem oder nach mehreren der vorhergehenden Ansprüche, wobei die Sammelvorrichtung (1) vor einer Verwendung in eine kompakte Form gefaltet ist, wobei die seitlichen Streifen (7) auf eine obere Seite des zentralen Streifens (6) entlang der Falten (8) zwischen jeder der zwei seitlichen Streifen (7) und dem zentralen Streifen (6) gefaltet sind.

7. Sammelvorrichtung nach einem oder nach mehreren der vorhergehenden Ansprüche, wobei der zentrale Streifen (6) ein hinteres Ende (18) aufweist, welches zwischen dem Stuhlproben-Sammelbereich (5) und dem hinteren Ende (4) der länglichen Schicht (2) eines faltbaren Materials angeordnet ist, so dass die längliche Schicht eines faltbaren Materials im Wesentlichen Y-förmig ist.

8. Sammelvorrichtung nach Anspruch 7, wobei sich die seitlichen Streifen (7) in einem Winkel zu einer Längsachse (15) des zentralen Streifens (6) in dem Bereich von 15-60 Grad, zum Beispiel in einem Winkel von 45 Grad, vorzugsweise in dem Bereich von 20-40 Grad, zum Beispiel in einem Winkel von 30 Grad erstrecken.

9. Sammelvorrichtung nach einem oder nach mehreren der vorhergehenden Ansprüche, wobei der zentrale Bereich (6) entlang seiner Länge eine konstante Breite in dem Bereich von 3 cm - 12 cm, zum Beispiel eine konstante Breite von 7 cm, aufweist.

10. Sammelvorrichtung nach einem oder nach mehreren der vorhergehenden Ansprüche, wobei die seitlichen Streifen (7) von dem hinteren Ende (4) der länglichen Schicht (2) eines faltbaren Materials zu dem vorderen Ende (3) der länglichen Schicht (2) eines faltbaren Materials verlaufen.

11. Sammelvorrichtung nach Anspruch 10, wobei die seitlichen Streifen (7) einen schmalen Mittelabschnitt aufweisen und sich zu ihrem hinteren Ende und zu ihrem vorderen Ende aufweiten.

12. Sammelvorrichtung nach Anspruch 10 oder Anspruch 11, wobei das vordere Ende (3) der länglichen Schicht (2) eines faltbaren Materials abgerundet ist, so dass es, wenn es während einer Verwendung auf einem Toilettensitz angebracht wird, im Wesentlichen mit dem Umfang eines Toilettensitzes (12) übereinstimmt.

13. Sammelvorrichtung nach einem oder nach mehreren der vorhergehenden Ansprüche, wobei die längliche Schicht (2) eines faltbaren Materials aus Papier und/oder biologisch abbaubaren Material hergestellt ist.

14. Sammelvorrichtung nach einem oder nach mehreren der vorhergehenden Ansprüche, wobei die längliche Schicht (2) eines faltbaren Materials ein Laminat ist, welches mehrere Schichten von verschiedenen Materialtypen umfasst.

15. Briefumschlag, welcher eine gefaltete Stuhlproben-Sammelvorrichtung nach einem oder nach mehreren der vorhergehenden Ansprüche umfasst.

## Revendications

1. Dispositif de collecte d'échantillon de selles (1) qui doit être monté sur le siège (12) des toilettes (13) pour recevoir un échantillon de selles d'une personne, le dispositif de collecte d'échantillon de selles (1) comprenant une feuille allongée (2) de matériau pliable prévue avec plusieurs tampons adhésifs (9) pour fixer la feuille allongée (2) sur le siège (12),
dans lequel la feuille allongée (2) s'étend dans une direction longitudinale entre une extrémité avant (3) et son extrémité arrière (4), et a une zone de collecte d'échantillon de selles (5) positionnée entre cette extrémité avant et cette extrémité arrière de la feuille allongée,
**caractérisé en ce que** :
la feuille allongée (2) comprend une bande centrale (6) et deux bandes latérales (7), laquelle bande centrale (6) s'étend dans la direction longitudinale de la feuille allongée (2), et lesquelles bandes latérales (7) sont positionnées de manière adjacente à et attenante à la bande centrale (6), et dans lequel un pli (8) est présent dans la feuille allongée (2) en tant que joint entre chaque bande latérale (7) et la bande centrale (6),
dans lequel la bande centrale (6) s'étend à partir de l'extrémité avant (3) de la feuille allongée (2) dans une direction arrière au-delà de la zone de collecte d'échantillon de selles (5), et les deux bandes latérales (7) s'étendent chacune à partir de l'extrémité arrière (4) de la feuille allongée (2) dans une direction avant au-delà de la zone de collecte d'échantillon de selles (5),
dans lequel la feuille allongée (2) en matériau pliable comprend une zone traversante de passage d'urine (10) entre l'extrémité avant (3) de la feuille allongée (2) et la zone de collecte d'échantillon de selles (5), dans laquelle zone traversante de passage d'urine (10), la feuille allongée en matériau pliable est prévue avec une ou plusieurs ouvertures traversantes de passage d'urine (11),
dans lequel au moins l'un de la pluralité de tampons adhésifs (9) est prévu sur un côté inférieur de la bande centrale (6) à proximité de l'extrémité avant (3) de la feuille allongée (2), et au moins l'un de la pluralité de tampons adhésifs (9) est prévu sur un côté inférieur de chacune des bandes latérales (7) à proximité de l'extrémité arrière (4) de la feuille allongée (2),
et dans lequel la feuille allongée (2) a une longueur de sorte que, lorsque le dispositif de collecte (1) est fixé avec l'extrémité avant (3) de la feuille allongée (2) sur une partie avant du siège (12) et avec l'extrémité arrière (4) de la feuille allongée (2) sur une partie arrière du siège (12), la zone de collecte d'échantillon de selles (5) est supportée au-dessous du siège (12) dans une cuvette de toilettes (13).

2. Dispositif de collecte selon la revendication 1, dans lequel la zone traversante de passage d'urine (10) comprend une rangée de plusieurs ouvertures allongées (11), ayant chacune un axe longitudinal s'étendant parallèlement à un axe longitudinal (15) de la feuille allongée (2).

3. Dispositif de collecte selon la revendication 1 ou la revendication 2, dans lequel la bande centrale (6) est en outre prévue avec une zone d'évacuation d'urine (16) positionnée entre la zone de collecte d'échantillon de selles (5) et la zone traversante de passage d'urine (1), laquelle zone d'évacuation d'urine s'étire au moins le long de la largeur de la bande centrale (6) et de préférence s'étire le long de la largueur des bandes latérales (7) également, laquelle zone d'évacuation d'urine (16) est prévue avec plusieurs ouvertures d'évacuation (17) pour évacuer l'urine s'écoulant le long de la bande centrale (6) de la zone traversante de passage d'urine (10) vers la zone de collecte d'échantillon de selles.

4. Dispositif de collecte selon la revendication 3, dans lequel la zone d'évacuation d'urine (16) comprend au moins deux rangées d'ouvertures d'évacuation (17), et dans lequel les ouvertures d'évacuation (17) d'une des au moins deux rangées sont disposées en quinconce par rapport aux ouvertures d'évacuation (17) de l'autre rangée.

5. Dispositif de collecte selon la revendication 3 ou la revendication 4, dans lequel les ouvertures d'évacuation (17) de la zone d'évacuation d'urine (16) sont sensiblement plus petites que l'ouverture traverse de passage d'urine (11) de la zone traversante de passage d'urine (10), de préférence la taille de chaque ouverture d'évacuation d'urine (17) représente au maximum 0,25 fois la taille des ouvertures traversantes de passage d'urine (10).

6. Dispositif de collecte selon une ou plusieurs des revendications précédentes, dans lequel le dispositif de collecte (1), avant l'utilisation, est plié en une forme compacte, dans laquelle les bandes latérales (7) sont pliées sur un côté supérieur de la bande centrale (6), le long des plis (8) entre chacune des deux bandes latérales (7) et la bande centrale (6) .

7. Dispositif de collecte selon une ou plusieurs des revendications précédentes, dans lequel la bande centrale (6) a une extrémité arrière (18) positionnée entre la zone de collecte d'échantillon de selles (5) et l'extrémité arrière (4) de la feuille allongée (2) de matériau pliable, de sorte que la feuille allongée en matériau pliable est sensiblement en forme de Y.

8. Dispositif de collecte selon la revendication 7, dans lequel les bandes latérales (7) s'étendent à un angle par rapport à un axe longitudinal (15) de la bande centrale (6) dans la plage de 15 à 60 degrés, par exemple à un angle de 45 degrés, de préférence dans la plage de 20 à 40 degrés, par exemple à un angle de 30 degrés.

9. Dispositif de collecte selon une ou plusieurs des revendications précédentes, dans lequel la bande centrale (6) a, le long de sa longueur, une largeur constante dans la plage de 3 cm à 12 cm, par exemple a une largeur constante de 7 cm.

10. Dispositif de collecte selon une ou plusieurs des revendications précédentes, dans lequel les bandes latérales (7) s'étendent à partir de l'extrémité arrière (4) de la feuille allongée (2) en matériau pliable jusqu'à l'extrémité avant (3) de la feuille allongée (2) en matériau pliable.

11. Dispositif de collecte selon la revendication 10, dans lequel les bandes latérales (7) ont une section centrale étroite, et s'évasent vers leur extrémité arrière et vers leur extrémité avant.

12. Dispositif de collecte selon la revendication 10 ou la revendication 11, dans lequel l'extrémité avant (3) de la feuille allongée (2) en matériau pliable est arrondie, de sorte que, lorsqu'elle est montée sur un siège de toilettes pendant l'utilisation, elle se couple sensiblement avec la circonférence d'un siège de toilettes (12).

13. Dispositif de collecte selon une ou plusieurs des revendications précédentes, dans lequel la feuille allongée (2) en matériau pliable est réalisée à partir de papier et/ou est réalisée avec un matériau biodégradable.

14. Dispositif de collecte selon une ou plusieurs des revendications précédentes, dans lequel la feuille allongée (2) en matériau pliable est un stratifié comprenant plusieurs feuilles de différents types de matériau.

15. Enveloppe postale comprenant un dispositif de collecte d'échantillon de selles plié selon une ou plusieurs des revendications précédentes.
